# EUROPÄISCHE PATENTSCHRIFT

(11) **EP 3 041 925 B1**
(45) Veröffentlichungstag und Bekanntmachung des Hinweises auf die Patenterteilung: **11.12.2019**
(21) Anmeldenummer: 14789774.8
(22) Anmeldetag: 18.08.2014
(51) Int. Cl.: C12M 1/107, C12M 1/00, C12M 1/06, C12M 1/34

(54) **VERFAHREN UND ANLAGE ZUR ERZEUGUNG VON BIOGAS**
METHOD AND PLANT FOR GENERATING BIOGAS
PROCÉDÉ ET INSTALLATION POUR LA GÉNÉRATION DE BIOGAZ

(30) Priorität: 05.09.2013 DE 102013014691; 28.02.2014 DE 102014002681
(43) Veröffentlichungstag der Anmeldung: 13.07.2016
(73) Patentinhaber: Envitec Biogas AG, 49393 Lohne (DE)
(72) Erfinder: TENBRINK, Jürgen, 48565 Steinfurt (DE)
(74) Vertreter: Siekmann, Gunnar
(86) Internationale Anmeldenummer: PCT/DE2014/000411
(87) Internationale Veröffentlichungsnummer: WO 2015/032375

(56) Entgegenhaltungen:
- WO-A1-2008/094282
- DE-A1-102008 061 461
- DE-A1-102009 058 588
- DE-A1-102010 043 630
- DE-U1-202013 102 153
- US-A- 4 869 819
- US-A1- 2011 033 908

## Beschreibung

Die Erfindung betrifft ein Verfahren zur Erzeugung von Biogas in einem mehrstufigen Abbauprozess mit wenigstens zwei hintereinander geschalteten Fermentern, bei dem Biomasse eines Ausgangssubstrates in einer ersten Abbaustufe in einem ersten Fermenter in verschiedene Zwischenprodukte, wie Kohlenstoff-dioxid, Wasserstoff, Alkohol, Essigsäure und andere organische Säuren, aufgespalten wird, die in einer der ersten Abbaustufe nachgeordneten, zweiten Abbaustufe in einem zweiten Fermenter zu dem Biogas verstoffwechselt werden, wobei wenigstens ein Teil des im ersten Fermenter als Zwischenprodukt anfallenden Kohlenstoffdioxids und Wasserstoffs als Wasserstoff-Kohlendioxid-Gemisch vor einer Methanisierung des Wasserstoff-Kohlenstoffdioxid-Gemisches aus dem Abbauprozess der Biogaserzeugung entfernt wird. Zudem betrifft die Erfindung eine Anlage zur Erzeugung von Biogas in einem mehrstufigen Abbauprozess mit wenigstens zwei hintereinander geschalteten Fermentern, denen jeweils eine Abbaustufe der Biogaserzeugung zugeordnet ist.

Der biologisch anaerobe Abbau von organischer Biomasse zu Biogas ist ein seit langem bekannter, natürlich ablaufender Prozess. Dieser erfolgt einerseits mit acetogenotrophen Bakterien über die Bildung von Essigsäure und andererseits mit hydrogenotrophen Bakterien über die Bildung von Wasserstoff und Kohlenstoffdioxid. Um diesen Abbauprozess in Biogasanlagen zu optimieren und die eingesetzte Biomasse möglichst effizient zu nutzen, wird zumeist versucht, durch eine geeignete Prozessführung und Biomassezusammensetzung sowie bauliche Maßnahmen an den Fermentern eine bessere Energieausbeute zu erzielen. Große Energieverluste sind jedoch schon dadurch bedingt, dass die Bildung von Methan aus Wasserstoff und Kohlenstoffdioxid durch die hydrogenotrophen Bakterien exotherm erfolgt und die dabei in Form von Wärme freigesetzte Energie für eine weitere Verwendung nicht nutzbar ist.

Ein Verfahren, um Energieverluste zu verringern, ist in der US 2011/0033908 A1 beschrieben. In dieser ist ein Fermenter offenbart, in dem bei einem Batch-Verfahren durch eine geeignete Prozessführung eines biologischen Abbauprozesses sequentiell zuerst Wasserstoff und nachfolgend Methan erzeugt werden soll. Der erzeugte Wasserstoff kann dann getrennt von dem Methan verwendet werden, so dass eine verbesserte Ausbeute erreichbar sein soll.

Aus der DE 10 2010 043 630 A1 geht ein Verfahren zur Erhöhung der Methankonzentration in Biogas und eine mehrstufige Anlage mit zwei hintereinander geschalteten Fermentern hervor, wobei in einen zweiten, einem ersten Fermenter nachgeschalteten Fermenter zusätzlich Wasserstoff eingeleitet wird, um einen höheren Methananteil im Biogas zu erreichen. Dieser Wasserstoff kann durch Aufreinigung eines aus dem ersten Fermenter abgeführten Wasserstoff-Kohlenstoffdioxid-Gemisches gewonnen werden, indem das Kohlenstoffdioxid aus dem Wasserstoff-Kohlenstoffdioxid-Gemisch entfernt wird. Der aufgereinigte Wasserstoff wird dann zusammen mit Hydrolysat aus dem ersten Fermenter in den zweiten Fermenter eingeleitet. Alternativ wird vorgeschlagen, dass dem zweiten Fermenter Wasserstoff aus einer externen Quelle zugeführt wird.

DE 10 2009 058588 offenbart ein Verfahren und eine Vorrichtung zur Herstellung von Biogas und beschreibt die Verwendung von dem Hauptfermenter nachgeschalteten Biogasspeicher und die Verwendung von Membrantrenneinrichtungen für die Gasseparation.

Aufgabe der Erfindung ist es, ein Verfahren und eine Anlage bereitzustellen, bei der die aus dem Abbau des Wasserstoffs und des Kohlenstoffdioxids resultierenden Energieverluste minimiert sind.

Die Lösung dieser Aufgabe erfolgt verfahrensmäßig mit den Merkmalen des Patentanspruchs 1 und vorrichtungsmäßig mit den Merkmalen des Patentanspruchs 11. Weiterbildungen und vorteilhafte Ausgestaltungen der jeweiligen Patentansprüche sind in den jeweiligen Unteransprüchen angegeben.

Das Verfahren zur Erzeugung von Biogas in einem mehrstufigen Abbauprozess mit wenigstens zwei hintereinander geschalteten Fermentern, bei dem Biomasse eines Ausgangssubstrates in einer ersten Abbaustufe in einem ersten Fermenter in verschiedene Zwischenprodukte, wie Kohlenstoffdioxid, Wasserstoff, Alkohol, Essigsäure und andere organische Säuren, aufgespalten wird, die in einer der ersten Abbaustufe nachgeordneten, zweiten Abbaustufe zu dem Biogas verstoffwechselt werden, wobei wenigstens ein Teil des im ersten Fermenter als Zwischenprodukt anfallenden Kohlenstoffdioxids und Wasserstoffs als Wasserstoff-Kohlenstoffdioxid-Gemisch vor einer Methanisierung des Wasserstoff-Kohlenstoffdioxid-Gemisches aus dem Abbauprozess der Biogaserzeugung entfernt wird, zeichnet sich erfindungsgemäß dadurch aus, dass das Wasserstoff-Kohlenstoffdioxid-Gemisch in einem getrennten Umwandlungsschritt katalytisch zu Methanol und Wasser oder Ameisensäure umgesetzt wird. Mit dem Methanol wird ein flüssiger, auf einfache Weise zu lagernder und zu transportierender Energieträger erzeugt, der entgegen dem bisher ausschließlich erzeugten Biogas auch vielseitiger einsetzbar ist. Bei Umwandlung zu Ameisensäure wird dementsprechend ein flüssiges, auf einfache Weise zu lagerndes und zu transportierender Produkt bereitgestellt. Da die Umwandlung des Kohlenstoffdioxids und des Wasserstoffs zu Methanol oder zu Ameisensäure aufgrund geringerer Energieverluste in Form von Wärme zudem effizienter abläuft, kann mit dem neuen, erfindungsgemäßen Verfahren in Form von Methanol beziehungsweise Ameisensäure und Biogas insgesamt mehr Energie bereitgestellt werden beziehungsweise der Rohstoff effektiver genutzt werden, als bei der alleinigen Erzeugung von Biogas, bei der Kohlenstoffdioxid und Wasserstoff exotherm zu Methan umgesetzt werden. Gemäß einer Weiterbildung können Methanol und Ameisensäure aber auch in zwei parallelen Prozessstufen oder in einem kombinierten Prozess umgesetzt werden.

Die Erzeugung des Biogases erfolgt dabei in einem Abbauprozess, bei dem das Substrat mit einer bestimmten Verweilzeit nacheinander durch den ersten und den zweiten Fermenter strömt und neues Substrat in den ersten Fermenter zugegeben wird während Produkte, insbesondere das Wasserstoff-Kohlenstoffdioxid-Gemisch der ersten Abbaustufe im ersten Fermenter und Methan der zweiten Abbaustufe im zweiten Fermenter, abgeführt werden.

Um das Wasserstoff-Kohlenstoffdioxid-Gemisch möglichst vollständig aus dem Abbauprozess der Biogaserzeugung entfernen zu können, muss sichergestellt werden, dass die Abbaustufen, das heißt die Aufspaltung in die Zwischenprodukte und die Methanisierung der Zwischenprodukte, möglichst vollständig getrennt voneinander ablaufen. Daraus folgt, dass eine Methanisierung des in der ersten Abbaustufe gebildeten Wasserstoffs und Kohlenstoffdioxids durch eine geeignete Prozessführung gehemmt werden sollte. Dies kann unter anderem dadurch erfolgen, dass die Methanisierung durch Einstellen einer Temperatur von weniger als 35° C, insbesondere circa 30° C, einen pH-Wert unter 7, insbesondere unter 6,5, und einer Verweilzeit von weniger als vier Tagen gehemmt wird. Die maximale Verweilzeit des Substrats von vier Tagen in der ersten Abbaustufe ist damit geringer als die Generationszeit der Methanbildner und sämtliche physikalische Parameter liegen außerhalb eines für Methanbildner optimalen mesophilen Temperatur- und pH-Bereichs, so dass eine Methanisierung des Wasserstoff-Kohlenstoffdioxid-Gemischs insgesamt effektiv unterbunden wird.

Die Verweilzeit der Biomasse in der ersten Abbaustufe kann in Abhängigkeit von dem zugeführten Ausgangssubstrat, das heißt vor allem dessen Menge und Zusammensetzung, über einen variablen Füllstand in der ersten Abbaustufe geregelt werden. Der Füllstand ist damit an verschiedene Mengen zugeführten Ausgangssubstrats anpassbar, wobei einer bestimmten Menge an zugeführtem Ausgangssubstrat ein bestimmter Füllstand der ersten Abbaustufe zugeordnet ist, der vorzugsweise zwischen 25% und 100 % eines Maximalfüllstands beträgt. Eine Anpassung des Füllstandes ist dabei insbesondere beim Anfahren, also zu Beginn des Abbauprozesses, erforderlich.

Da sich insbesondere der Wasserstoff durch dessen Anreicherung in dem Substrat auch hemmend auf weitere Abbauprozesse auswirken kann, sollte einer entsprechenden Anreicherung in der Abbaustufe entgegengewirkt werden. Das Wasserstoff-Kohlenstoffdioxid-Gemisch kann dazu mittels Unterdruck aus dem Abbauprozess der Biogaserzeugung entfernt werden.

Weiterhin kann das Substrat alternativ oder ergänzend zu einem anzulegenden Unterdruck auch durch intensives Rühren entgast und ein niedriger Partialdruck des Wasserstoffs in dem Substrat erreicht werden. Die erste Abbaustufe der Biogaserzeugung wird daher vorteilhafterweise volldurchmischt, während die zweite Abbaustufe in einem nicht volldurchmischten Aufstromverfahren betrieben wird. Entgegen der ersten Abbaustufe, bei der eine möglichst effektive Entgasung der Biomasse erfolgen soll, wird die zweite Abbaustufe damit nur in einzelnen Ebenen gerührt, ohne dass diese untereinander durchmischt werden. Dadurch ist eine möglichst lange Verweilzeit der Biomasse und eine gute Biomasserückhaltung in der zweiten Abbaustufe gewährleistet. Weiter sind Ausschwemmverluste und Kurzschlussströmungen effektiv unterbunden, so dass ein möglichst vollständiger Abbau der Alkohole und/oder organischen Säuren erreicht ist. Eine hohe Ausbeute in der zweiten Abbaustufe kann zudem durch höhere Temperaturen von ca. 55 °C und einem pH-Wert von 7 bis 8 sichergestellt werden.

Je nach Zusammensetzung des Substrats weist das aus dem Abbauprozess der Biogaserzeugung entfernte Wasserstoff-Kohlenstoffdioxid-Gemisch ein unterschiedliches Mengenverhältnis zwischen dem Kohlenstoffdioxid und dem Wasserstoff auf, so dass das Wasserstoff-Kohlenstoffdioxid-Gemisch zumeist nicht vollständig in Methanol oder Ameisensäure umgewandelt werden kann. Nach einer Weiterbildung der Erfindung ist daher vorgesehen, dass nicht zu Methanol oder zu Ameisensäure umgesetzter Wasserstoff und/oder nicht umgesetztes Kohlenstoffdioxid zusammen mit aus der zweiten Abbaustufe der Biogaserzeugung gewonnenem Biogas gespeichert wird. Nicht zu Methanol oder zu Ameisensäure umgesetzter Wasserstoff kann dann zusammen mit dem Biogas energetisch genutzt werden, indem ein Blockheizkraftwerk für eine entsprechende Mischgasnutzung modifiziert und ausgelegt wird. Alternativ kann das überschüssige Wasserstoff-Kohlenstoffdioxid-Gemisch auch der zweiten Abbaustufe zugeführt werden und auf herkömmliche Weise verstoffwechselt werden.

Um nur geringe Mengen an überschüssigem Wasserstoff beziehungsweise Kohlenstoffdioxid nach dem Katalysieren des Methanols oder der Ameisensäure verwerten zu müssen und eine hohe Ausbeute zu erreichen, kann in Abhängigkeit von der Zusammensetzung des Wasserstoff-Kohlenstoffdioxid-Gemisches zudem vorgesehen sein, dass dem katalytisch zu Methanol oder zu Ameisensäure umzusetzenden Wasserstoff-Kohlenstoffdioxid-Gemisch, bei einem Wasserstoffüberschuss Kohlenstoff-dioxid, insbesondere Kohlenstoffdioxid aus Abgas, zugeführt wird. Quelle für das Abgas kann beispielsweise ein Blockheizkraftwerk sein, in dem das Biogas der zweiten Abbaustufe energetisch genutzt wird und das mit der katalytischen Methanol- oder Ameisensäureerzeugung gekoppelt ist. Bei einem Kohlenstoffdioxid-überschuss kann erfindungsgemäß vorgesehen sein, dass dem katalytisch zu Methanol oder zu Ameisensäure umzusetzenden Wasserstoff-Kohlenstoffdioxid-Gemisch mittels Elektrolyse gewonnener Wasserstoff zugeführt wird. Sowohl der zugeführte Wasserstoff als auch das Abgas mit dem Kohlenstoffdioxid sorgen dann jeweils für eine höhere Ausbeute an Methanol oder Ameisensäure im Gesamtprozess.

Weiter betrifft die Erfindung eine Anlage zur Biogaserzeugung in einem mehrstufigen Abbauprozess mit wenigstens zwei hintereinander geschalteten Fermentern, denen jeweils eine Abbaustufe der Biogaserzeugung zugeordnet ist. Diese Anlage zeichnet sich erfindungsgemäß dadurch aus, dass der Fermenter, dem eine erste Abbaustufe zu Zwischenprodukten, wie Kohlenstoffdioxid, Wasserstoff, Alkohol, Essigsäure und anderen organischen Säuren zugeordnet ist, eine Gasentnahmeeinrichtung aufweist, und dass die Gasentnahmeeinrichtung über eine Entnahmeleitung mit wenigstens einem Katalysator verbunden ist. Die bei der Biogaserzeugung als Zwischenprodukte entstehenden Gase Kohlenstoffdioxid und Wasserstoff werden dann als Wasserstoff-Kohlenstoffdioxid-Gemisch über die Gasentnahmeeinrichtung und die Entnahmeleitung, die auch in einem Bauteil zusammengefasst sein können, dem Katalysator zugeführt. Dieser Katalysator ist in weiterer Ausgestaltung ein Methanolkatalysator oder Ameisensäurekatalysator, je nach dem, ob das Wasserstoff-Kohlenstoffdioxid-Gemisch zu Methanol oder zu Ameisensäure umgesetzt werden soll. Weiter können auch ein Methanolkatalysator und ein Ameisensäurekatalysator in einer Prozessstufe vorgesehen sein, so dass mit einer Anlage Methanol und Ameisensäure bereitgestellt werden können. In diesem wird das Wasserstoff-Kohlenstoffdioxid-Gemisch anstatt zu Methan in Methanol oder in Ameisensäure umgewandelt, während die restlichen Zwischenprodukte, wie Alkohol, Essigsäure und andere organische Säuren, auf herkömmliche Weise zu Biogas verstoffwechselt werden. Durch die Abtrennung des Wasserstoff-Kohlenstoffdioxid-Gemisches und dessen Umwandlung zu Methanol oder zu Ameisensäure, kann aus der Biomasse insgesamt mehr und höherwertige Energie gewonnen werden, da weniger Energie in Form von Wärme freigesetzt wird und das Methanol oder die Ameisensäure vielseitiger einsetzbar sowie einfacher zu handhaben ist, das heißt unter anderem gelagert und transportiert werden kann.

Um einen guten, das heißt schnellen Gasaustrag aus dem Fermenter mit der Gasentnahmeeinrichtung zu gewährleisten, kann dieser ein diesen volldurchmischendes Rührwerk aufweisen. In weiterer Ausgestaltung kann dieser Fermenter liegend mit einer länglichen, insbesondere zylindrischen Grundform ausgebildet sein und ein Rührwerk mit einem oder mehreren Rührorganen aufweisen.

Neben oder anstatt eines Rührwerks, mit dem der Fermenter mit der Gasentnahmeeinrichtung volldurchmischbar ist, kann gemäß einer Weiterbildung vorgesehen sein, dass die Gasentnahmeeinrichtung einen Unterdruckerzeuger aufweist. Sowohl mit dem Rührwerk als auch mit dem Unterdruckerzeuger, beispielsweise einer in der Entnahmeleitung angeordneten Vakuumpumpe, können sich hemmend auf den weiteren Abbau der Biomasse auswirkende Partialdrücke von beim Abbau entstehenden Gasen in dem abzubauenden Substrat verringert werden.

Weiter kann der Fermenter, in dem die Biomasse in die Zwischenprodukte Kohlenstoffdioxid, Wasserstoff, Alkohol und organische Säuren aufgespalten wird, eine umfangreiche Füllstandserfassung und -steuerung aufweisen, mit der unter anderem in den Fermenter eingetragenes Ausgangssubstrat und der Füllstand in dem Fermenter selbst erfassbar ist. Anhand des Füllstandes in dem Fermenter und des in den Fermenter eingetragenen Ausgangssubstrats ist dann durch Regeln des Füllstandes in dem Fermenter eine vorbestimmte Verweilzeit der Biomasse einstellbar.

Von dem Katalysator kann wenigstens eine Gasspeicherleitung abgehen, die in einen gemeinsamen, den Fermentern zur Biogaserzeugung nachgestalteten Gasspeicher einmündet. Über diese Gasspeicherleitung kann dann nicht katalytisch umgesetztes Wasserstoff-Kohlenstoffdioxid-Gemisch abgeführt und gemeinsam mit dem erzeugten Biogas gespeichert werden. Das Biogas sowie das zugeführte Wasserstoff-Kohlenstoffdioxid-Gemisch können dann zusammen in einem modifizierten Mischgas-Blockheizkraftwerk energetisch genutzt werden. Für die energetische Nutzung wird demnach mit Vorteil in der gesamten Anlage nur dieses eine Mischgas-Blockheizkraftwerk benötigt. Gemäß einer ersten alternativen Ausgestaltung der Anlage könnte auch ein zweites Blockheizkraftwerk vorgesehen werden, so dass von dem Katalysator abgeführter Wasserstoff und in den Fermentern umgesetztes Biogas getrennt voneinander energetisch genutzt werden.

Um eine höhere Methanol- oder Ameisensäureausbeute zu erzielen, kann dem Katalysator zudem eine Elektrolyse zugeordnet sein und/oder der Katalysator derart mit dem Mischgas-Blockheizkraftwerk oder einer anderen kohlenstoffdioxidhaltigen Abgasquelle verschaltet sein, dass je nachdem ob Wasserstoff oder Kohlenstoffdioxid im Wasserstoff-Kohlenstoffdioxid-Gemisch im Überschuss vorliegt, Wasserstoff von der Elektrolyse oder Kohlenstoffdioxid der Abgasquelle zuführbar ist.

Ein Ausführungsbespiel der Erfindung, aus dem sich weitere erfinderische Merkmale ergeben können, ist in der Zeichnung dargestellt. Es zeigen:
- Fig. 1:: ein Verfahrensschema einer erfindungsgemäßen Anlage zur Biogaserzeugung;
- Fig. 2:: einen ersten Fermenter der erfindungsgemäßen Anlage zur Biogaserzeugung gemäß Fig. 1; und
- Fig. 3:: einen zweiten Fermenter der Anlage zur Biogaserzeugung gemäß Fig.1.

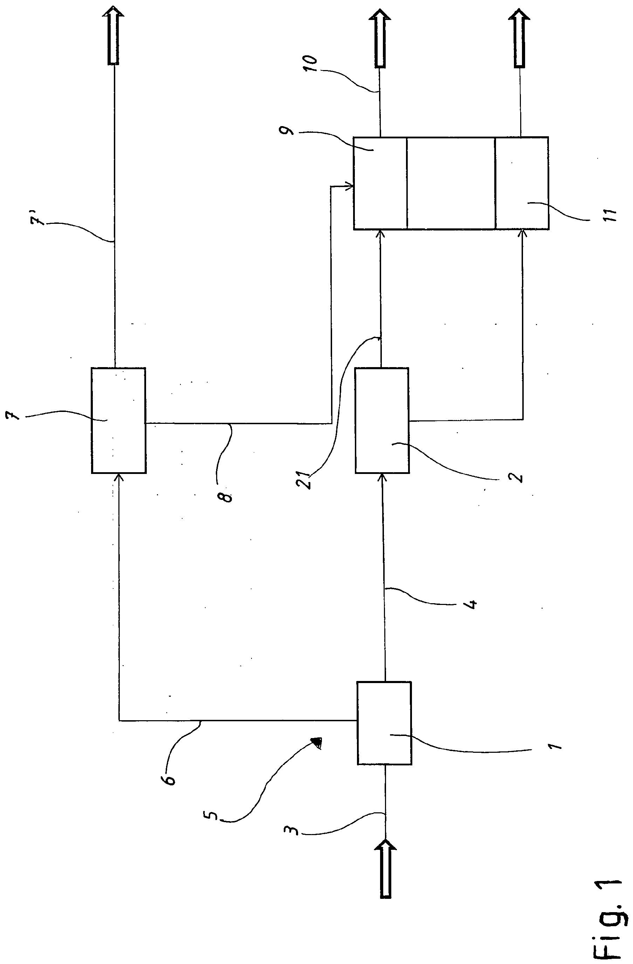

In Fig. 1 ist eine Anlage zur Biogaserzeugung mit einem ersten Fermenter 1 und einem dem ersten Fermenter 1 nachgeordneten zweiten Fermenter 2 dargestellt. Dem Fermenter 1 ist ein Materialeintrag 3 zugeordnet, über den Biomasse in die Anlage förderbar ist. In dem Fermenter 1 erfolgt dann eine erste Aufspaltung der Biomasse zu Zwischenprodukten. Zwischen den Fermentern 1, 2 ist eine Substratleitung 4 ausgebildet, über die in dem ersten Fermenter 1 gebildete flüssige und feste Zwischenprodukte in den zweiten Fermenter 2 überführbar sind. Über eine dem Fermenter 1 zugeordnete Gasentnahmeeinrichtung 5 und Entnahmeleitung 6 sind gasförmige Zwischenprodukte, insbesondere Wasserstoff-Kohlenstoffdioxid-Gemisch aus dem Fermenter 1 zu einen an die Entnahmeleitung 6 angeschlossenen Katalysator 7 überführbar. In dem Katalysator 7 erfolgt dann eine Umwandlung der gasförmigen Zwischenprodukte, insbesondere Wasserstoff und Kohlenstoffdioxid bei einem Methanolkatalysator zu Methanol und Wasser oder bei einem Ameisenkatalysator zu Ameisensäure. Das Methanol oder die Ameisensäure ist über eine Leitung 7' des Katalysators 7 abführbar. Überschüssiges, nicht zu Methanol und Wasser oder zu Ameisensäure umgesetztes Wasserstoff-Kohlenstoffdioxid-Gemisch ist über eine Gasspeicherleitung 8 einem Gasspeicher 9 zuführbar, in dem auch in dem zweiten Fermenter 2 gewonnenes Biogas zwischenspeicherbar ist. Das aus dem Wasserstoff-Kohlenstoffdioxid-Gemisch und dem Biogas bestehende Mischgas kann dann über einen entsprechenden Gasspeicheranschluss 10 einer energetischen Nutzung in einem Mischgas-Blockheizkraftwerk zugeführt werden. Feste beziehungsweise flüssige Bestandteile, die nicht zu Biogas umgesetzt wurden, werden von dem zweiten Fermenter 2 in einen Gärrestspeicher 11 überführt und können als Dünger genutzt werden.

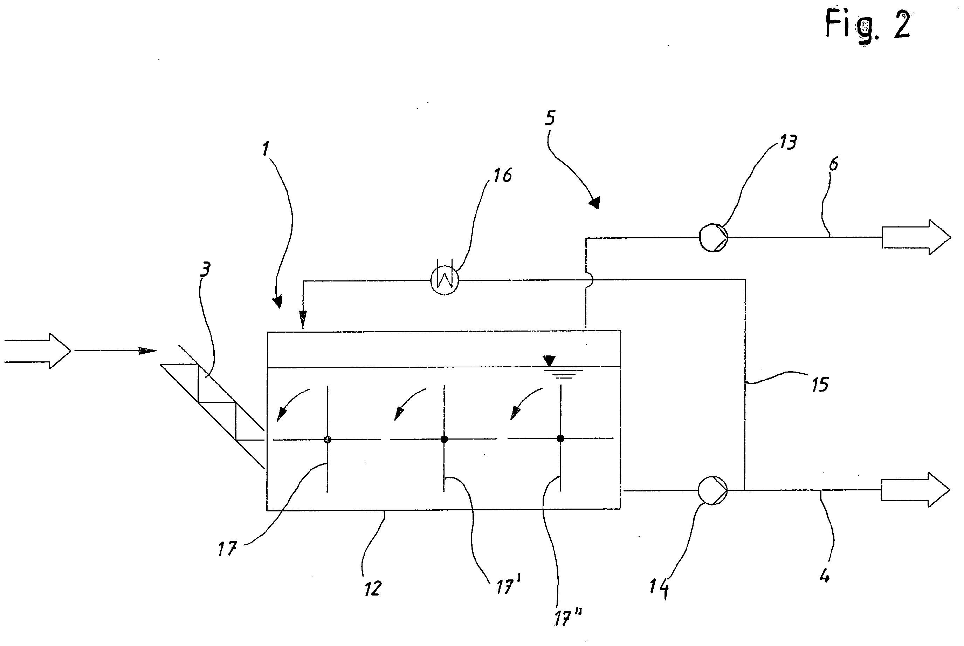

In Fig. 2 ist der Fermenter 1 im Detail dargestellt. Dieser besteht aus einem länglichen, liegend angeordneten Behälter 12, an den der Materialeintrag 3 sowie die Gasentnahmeeinrichtung 5 mit der Entnahmeleitung 6 sowie einem als Vakuumpumpe ausgebildeten Unterdruckerzeuger 13 angeschlossen ist. Weiter geht von dem Behälter 12 die Substratleitung 4 ab, der eine Substratpumpe 14 zugeordnet ist. In Strömungsrichtung hinter der Substratpumpe 14 zweigt von der Substratleitung 4 eine Rückführleitung 15 ab, über die ein Teilstrom des flüssigen Substrats über einen ersten Wärmetauscher 16 wieder in den Behälter 12 rückführbar ist. In dem Behälter 12 selbst sind mehrere Rührorgane 17, 17', 17" eines den Fermenter 1 volldurchmischenden Rührwerks angeordnet.

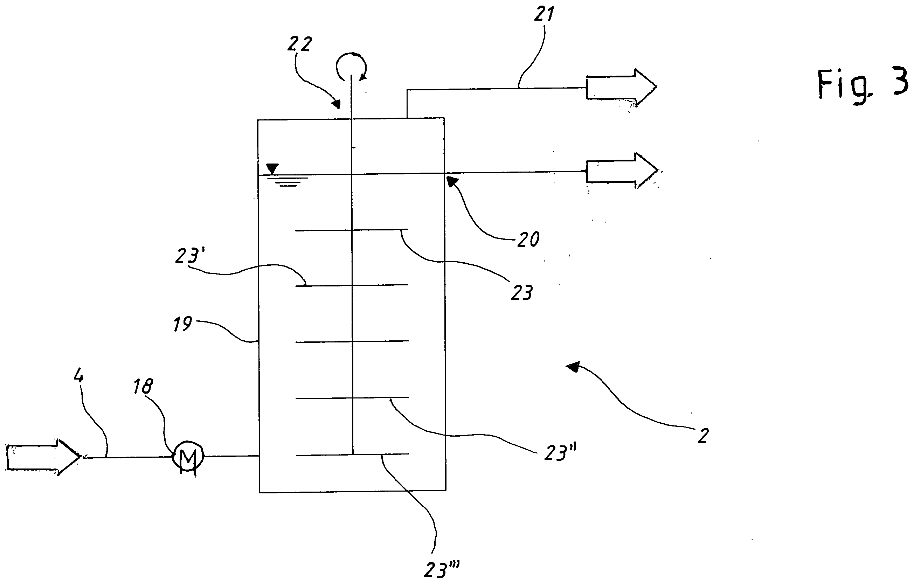

Fig. 3 zeigt den zweiten Fermenter 2 in den die über einen weiteren Wärmetauscher 18 geführte Substratleitung 4 in einen unteren Bereich des Fermenters 2 einmündet. Der Fermenter 2 ist als Aufstromfermenter ausgebildet und weist einen Behälter 19 auf, der in Längsrichtung von unten nach oben durchströmbar ist und in einem oberen Bereich einen zu dem Gärrestspeicher 11 führenden Überlauf 20 sowie eine zu dem Gasspeicher 9 führende Gasspeicherleitung 21 aufweist. Um den Behälter 19 zu durchmischen, weist dieser ein Rührwerk 22 mit in jeweils einzelnen Ebenen rührenden Rührorganen 23, 23', 23", 23''' auf.

## Patentansprüche

1. Verfahren zur Erzeugung von Biogas in einem mehrstufigen Abbauprozess mit wenigstens zwei hintereinander geschalteten Fermentern, bei dem Biomasse eines Ausgangssubstrates in einer ersten Abbaustufe in einem ersten Fermenter in verschiedene Zwischenprodukte, wie Kohlenstoffdioxid, Wasserstoff, Alkohol, Essigsäure und andere organische Säuren, aufgespalten wird, die in einer der ersten Abbaustufe nachgeordneten, zweiten Abbaustufe in einem zweiten Fermenter zu dem Biogas verstoffwechselt werden, wobei wenigstens ein Teil des im ersten Fermenter als Zwischenprodukt anfallenden Kohlenstoffdioxids und Wasserstoffs als Wasserstoff-Kohlenstoffdioxid-Gemisch vor einer Methanisierung des Wasserstoff-Kohlenstoffdioxid-Gemisches aus dem Abbauprozess der Biogaserzeugung entfernt wird,
**dadurch gekennzeichnet,**
**dass** das Wasserstoff-Kohlenstoffdioxid-Gemisch in einem getrennten Umwandlungsschritt katalytisch zu Methanol und Wasser oder zu Ameisensäure umgesetzt wird.

2. Verfahren nach Anspruch 1, **dadurch gekennzeichnet, dass** eine Methanisierung des in der ersten Abbaustufe gebildeten Wasserstoffs und Kohlenstoffdioxids durch eine geeignete Prozessführung gehemmt ist.

3. Verfahren nach Anspruch 2, **dadurch gekennzeichnet, dass** die Methanisierung durch Einstellen einer Temperatur von weniger als 35°C, insbesondere circa 30°C, einem pH-Wert unter 7, insbesondere unter 6,5, und einer Verweilzeit von weniger als vier Tagen gehemmt wird.

4. Verfahren nach Anspruch 3, **dadurch gekennzeichnet, dass** die Verweilzeit der Biomasse in der ersten Abbaustufe in Abhängigkeit von dem zugeführten Ausgangssubstrat über einen variablen Füllstand in der ersten Abbaustufe geregelt wird.

5. Verfahren nach einem der Ansprüche 1 bis 4, **dadurch gekennzeichnet, dass** das Wasserstoff-Kohlenstoffdioxid-Gemisch mittels Unterdruck aus dem Abbauprozess der Biogaserzeugung entfernt wird.

6. Verfahren nach einem der Ansprüche 1 bis 5, **dadurch gekennzeichnet, dass** die erste Abbaustufe der Biogaserzeugung volldurchmischt wird, während die zweite Abbaustufe als nicht volldurchmischtes Aufstromverfahren betrieben wird.

7. Verfahren nach einem der Ansprüche 1 bis 6, **dadurch gekennzeichnet, dass** nicht zu Methanol oder zu Ameisensäure umgesetzter Wasserstoff und/oder nicht zu Methanol oder zu Ameisensäure umgesetzter Kohlenstoffdioxid des Wasserstoff-Kohlenstoffdioxid-Gemisches zusammen mit aus der zweiten Abbaustufe der Biogaserzeugung gewonnenem Biogas gespeichert wird.

8. Verfahren nach Anspruch 7, **dadurch gekennzeichnet, dass** nicht umgesetzter Wasserstoff zusammen mit dem aus der zweiten Abbaustufe der Biogaserzeugung gewonnenem Biogas energetisch genutzt wird.

9. Verfahren nach einem der Ansprüche 1 bis 8, **dadurch gekennzeichnet, dass** dem katalytisch zu Methanol oder zu Ameisensäure umzusetzenden Wasserstoff-Kohlenstoffdioxid-Gemisch bei einem Wasserstoffüberschuss Kohlenstoffdioxid, insbesondere Abgas, zugeführt wird.

10. Verfahren nach einem der Ansprüche 1 bis 9, **dadurch gekennzeichnet, dass** dem katalytisch zu Methanol oder zu Ameisensäure umzusetzenden Wasserstoff-Kohlenstoffdioxid-Gemisch bei einem Kohlenstoffdioxid-überschuss mittels Elektrolyse gewonnener Wasserstoff zugeführt wird.

11. Anlage zur Biogaserzeugung in einem mehrstufigen Abbauprozess mit wenigstens zwei hintereinander geschalteten Fermentern (1, 2), denen jeweils eine Abbaustufe der Biogaserzeugung zugeordnet ist,
**dadurch gekennzeichnet,**
**dass** der Fermenter (1), dem eine erste Abbaustufe zu Zwischenprodukten, wie Kohlenstoffdioxid, Wasserstoff, Alkohol, Essigsäure und anderen organischen Säuren, zugeordnet ist, eine Gasentnahmeeinrichtung (5) aufweist, und
**dass** die Gasentnahmeeinrichtung (5) über eine Entnahmeleitung (6) mit wenigstens einem Katalysator (7) verbunden ist.

12. Anlage nach Anspruch 11, **dadurch gekennzeichnet, dass** der Katalysator (7) ein Methanolkatalysator ist.

13. Anlage nach einem der Ansprüche 11 oder 12, **dadurch gekennzeichnet, dass** der Katalysator (7) ein Ameisensäurekatalysator ist.

14. Anlage nach einem der Ansprüche 11 bis 13, **dadurch gekennzeichnet, dass** die Gasentnahmeeinrichtung (5) einen Unterdruckerzeuger (13) aufweist.

15. Anlage nach einem der Ansprüche 11 bis 14, **dadurch gekennzeichnet, dass** dem Fermenter (1) mit der Gasentnahmeeinrichtung (5) ein nicht volldurchmischter Aufstromfermenter nachgeschaltet ist.

16. Anlage nach einem der Ansprüche 11 bis 15, **dadurch gekennzeichnet, dass** von dem Katalysator (7) wenigstens eine Gasspeicherleitung (8) abgeht, die in einen gemeinsamen, den Fermentern (1, 2) nachgeschalteten Gasspeicher (9) einmündet.

## Claims

1. A method for producing biogas in a multi-stage decomposition process with at least two consecutively connected fermenters, in which biomass from a starting substrate is split into various intermediate products such as carbon dioxide, hydrogen, alcohol, acetic acid and other organic acids in a first decomposition stage in a first fermenter, which products are metabolised to yield the biogas in a second decomposition stage in the second fermenter which is arranged downstream of the first decomposition stage, wherein at least some of the carbon dioxide and hydrogen produced as an intermediate product in the first fermenter is removed from the decomposition process for producing biogas as a hydrogen-carbon dioxide mixture prior to methanation of the hydrogen-carbon dioxide mixture,
**characterized in that**
the hydrogen-carbon dioxide mixture is catalytically converted to methanol and water or formic acid in a separate conversion step.

2. The method according to claim 1, **characterized in that** a methanation of the hydrogen and carbon dioxide formed in the first decomposition process is inhibited by suitable process control.

3. The method according to claim 2, **characterized in that** the methanation is inhibited by setting a temperature below 35 °C, in particular about 30 °C, a pH value lower than 7, in particular lower than 6.5, and a residence time of less than four days.

4. The method according to claim 3, **characterized in that** the residence time of the biomass in the first decomposition stage is controlled via a variable fill level in the first decomposition stage depending on the supplied starting substrate.

5. The method according to any one of claims 1 to 4,
**characterized in that** the hydrogen-carbon dioxide mixture is removed from the decomposition process of the biogas production by means of negative pressure.

6. The method according to any one of claims 1 to 5, **characterized in that** the first decomposition stage of biogas production is fully mixed while the second decomposition stage is operated as a non-fully mixed upward flow process.

7. The method according to any one of claims 1 to 6,
**characterized in that** hydrogen of the hydrogen-carbon dioxide mixture which is not transformed into methanol or formic acid, and/or the carbon dioxide of the hydrogen-carbon dioxide mixture which is not transformed into methanol or formic acid is stored together with biogas that is recovered from the second decomposition stage of the biogas production.

8. The method according to claim 7, **characterized in that** unreacted hydrogen is used together with the biogas recovered from the second decomposition stage of biogas production for energy purposes.

9. The method according to any one of claims 1 to 8,
**characterized in that** carbon dioxide with an excess of hydrogen, particularly exhaust gas, is added to the hydrogen-carbon dioxide mixture which is to be transformed catalytically into methanol or formic acid.

10. The method according to any one of claims 1 to 9,
**characterized in that** hydrogen with an excess of carbon dioxide which is recovered by means of electrolysis is added to the hydrogen-carbon dioxide mixture which is to be transformed catalytically into methanol or formic acid.

11. A plant for producing biogas in a multi-stage decomposition process with at least two consecutively connected fermenters (1, 2), to each of which a decomposition stage for biogas production is assigned,
**characterized**
**in that** the fermenter (1) to which a first stage of decomposition to intermediate products such as carbon dioxide, hydrogen, alcohol, acetic acid and other organic acids is assigned, is equipped with a gas extraction system (5), and
**in that** the gas extraction system (5) is connected to at least one catalyst (7) via an extraction line (6).

12. The plant according to claim 11, **characterized in that** the catalyst (7) is a methanol catalyst.

13. The plant according to claim 11 or 12, **characterized in that** the catalyst (7) is a formic acid catalyst.

14. The plant according to any one of claims 11 to 13,
**characterized in that** the gas extraction system (5) is equipped with a vacuum generator (13).

15. The plant according to any one of claims 11 to 14,
**characterized in that** a non-fully mixed upflow fermenter is connected downstream of the fermenter (1) with the gas extraction system (5).

16. The plant according to any one of claims 11 to 15,
**characterized in that** at least one gas storage line (8) leaves the catalyst (7) and discharges into a shared gas storage means (9) which is connected downstream of the fermenters (1, 2).

## Revendications

1. Procédé, destiné à produire du biogaz au cours d'un processus de dégradation en plusieurs étapes, à l'aide d'au moins deux digesteurs montés l'un derrière l'autre, lors duquel dans une première étape de dégradation, de la biomasse d'un substrat initial est décomposée dans un premier digesteur en différents produits intermédiaires, tels que le dioxyde de carbone, l'hydrogène, l'alcool, l'acide acétique et d'autres acides organiques, qui dans une deuxième étape de dégradation ayant lieu en aval de la première étape de dégradation sont métabolisés dans un deuxième digesteur pour obtenir le biogaz, au moins une partie du dioxyde de carbone et de l'hydrogène résultant dans le premier digesteur en tant que produit intermédiaire étant retirée du processus de dégradation de la production du biogaz sous la forme d'un mélange hydrogène/dioxyde de carbone, avant une méthanisation du mélange hydrogène/dioxyde de carbone,
**caractérisé en ce que**
dans une étape de transformation séparée, le mélange hydrogène/dioxyde de carbone est catalytiquement transformé en méthanol et en eau ou en acide formique.

2. Procédé selon la revendication 1, **caractérisé en ce qu'**une méthanisation de l'hydrogène et du dioxyde de carbone formés lors de la première étape de dégradation est inhibée par une gestion adaptée du processus.

3. Procédé selon la revendication 2, **caractérisé en ce que** la méthanisation est inhibée par réglage d'une température inférieure à 35 °C, notamment d'environ 30 °C, d'une valeur pH inférieure à 7, notamment inférieure à 6,5, et d'un temps de séjour inférieur à quatre jours.

4. Procédé selon la revendication 3, **caractérisé en ce que** le temps de séjour de la biomasse dans la première étape de dégradation est réglé dans la première étape de dégradation, en fonction du substrat initial alimenté, par l'intermédiaire d'un niveau de remplissage variable.

5. Procédé selon l'une quelconque des revendications 1 à 4, **caractérisé en ce que** le mélange hydrogène/dioxyde de carbone est retiré par dépression du processus de dégradation de la production de biogaz.

6. Procédé selon l'une quelconque des revendications 1 à 5, **caractérisé en ce que** la première étape de dégradation de la production de biogaz est totalement malaxée, alors que la deuxième étape de dégradation est exploitée sous la forme d'un procédé à flux ascensionnel non totalement malaxé.

7. Procédé selon l'une quelconque des revendications 1 à 6, **caractérisé en ce que** l'hydrogène non transformé en méthanol ou en acide formique et/ou le dioxyde de carbone non transformé en méthanol ou en acide formique du mélange hydrogène/dioxyde de carbone est accumulé conjointement avec le biogaz obtenu à partir de la deuxième étape de dégradation de la production de biogaz.

8. Procédé selon la revendication 7, **caractérisé en ce que** de l'hydrogène non transformé est utilisé sous forme énergétique, conjointement avec le biogaz obtenu à partir de la deuxième étape de dégradation de la génération de biogaz.

9. Procédé selon l'une quelconque des revendications 1 à 8, **caractérisé en ce que**, dans le cas d'un excédent d'hydrogène, le mélange hydrogène/dioxyde de carbone qui doit être transformé catalytiquement en méthanol ou en acide formique est alimenté en dioxyde de carbone, notamment en gaz d'échappement.

10. Procédé selon l'une quelconque des revendications 1 à 9, **caractérisé en ce que**, dans le cas d'un excédent de dioxyde de carbone, le mélange hydrogène/dioxyde de carbone qui doit être transformé catalytiquement en méthanol ou en acide formique est alimenté en hydrogène, produit par électrolyse.

11. Installation, destinée à produire du biogaz au cours d'un processus de dégradation en plusieurs étapes, à l'aide d'au moins deux digesteurs (1, 2) montés l'un derrière l'autre, chacun d'eux étant affecté à une étape de dégradation de la production de biogaz.
**caractérisée**
**en ce que** le digesteur (1), auquel est associé une première étape de dégradation en produits intermédiaires, tels que le dioxyde de carbone, l'hydrogène, l'alcool, l'acide acétique et d'autres acides organiques comporte un système de prélèvement gazeux (5) et
**en ce que** le système de prélèvement gazeux (5) est relié par l'intermédiaire d'au moins un conduit de prélèvement (6) avec au moins un catalyseur (7).

12. Installation selon la revendication 11, **caractérisé en ce que** le catalyseur (7) est un catalyseur de méthanol.

13. Installation selon l'une quelconque des revendications 11 ou 12, **caractérisé en ce que** le catalyseur (7) est un catalyseur d'acide formique.

14. Installation selon l'une quelconque des revendications 11 à 13, **caractérisé en ce que** le système de prélèvement gazeux (5) comporte un générateur de dépression (13).

15. Installation selon l'une quelconque des revendications 11 à 14, **caractérisé en ce qu'**en aval du digesteur (1) pourvu du système de prélèvement gazeux (5) est monté un digesteur à flux ascendant, non totalement malaxé.

16. Installation selon l'une quelconque des revendications 11 à 15, **caractérisé en ce qu'**au moins un conduit accumulateur de gaz (8) qui débouche dans un accumulateur de gaz (9) commun, monté en aval des digesteurs (1, 2) part du catalyseur (7).
